# EUROPEAN PATENT APPLICATION

(11) **EP 4 227 405 A1**
(43) Date of publication of application: **16.08.2023**
(21) Application number: 21877593.0
(22) Date of filing: 05.10.2021
(51) Int. Cl.: C12N 5/0775, A61K 35/28, A61P 19/02

(54) **METHOD FOR PRODUCING CELL FORMULATION FOR JOINT TREATMENT, CELL FORMULATION FOR JOINT TREATMENT, AND METHOD FOR CULTURING MESENCHYMAL STEM CELLS**

(30) Priority: 07.10.2020 JP 2020169712; 28.01.2021 JP 2021011736
(71) Applicant: FUJIFILM Corporation, Tokyo 106-8620 (JP)
(72) Inventor: NAKAMURA, Kentaro, Kanagawa 258-8577 (JP); YOSHINO, Yuichi, Kanagawa 258-8577 (JP); INADA, Atsushi, Kanagawa 258-8577 (JP); KITAHASHI, Tsukasa, Kanagawa 258-8577 (JP)
(74) Representative: Hoffmann Eitle
(86) International application number: PCT/JP2021/036723
(87) International publication number: WO 2022/075294

(57) **Abstract**

An object of the present invention is to provide a method of producing a cell preparation for a joint medical treatment, in which a cell proliferation ratio can be improved, an amount of serum required can be reduced, and karyotype abnormalities in a culture process can be suppressed, a method of culturing a mesenchymal stem cell, and a cell preparation for a joint medical treatment, which is produced by the above method. According to the present invention, there is provided a method of producing a cell preparation for a joint medical treatment, the method including culturing a mesenchymal stem cell in a culture medium containing an ascorbic acid-2-phosphate trisodium salt, alanyl glutamine, and pyridoxine.

## Description

### Field of the Invention

The present invention relates to a method of producing a cell preparation for a joint medical treatment and a method of culturing a mesenchymal stem cell, which include culturing a mesenchymal stem cell in a culture medium containing a predetermined component. The present invention further relates to a cell preparation for a joint medical treatment, which is produced by the above method.

### Description of the Related Art

In the field of orthopedics, articular cartilage injury and meniscus injury are frequently seen as objects of daily medical practice and are widely recognized as diseases from which a large number of patients suffer. In a case where articular cartilage injury or meniscus injury occurs, arthralgia, a decrease in a mobile region, hydrarthrosis, dyskinesia, or the like occurs. A patient having traumatic articular cartilage injury or meniscus injury is generally treated medically by an orthopaedist. Surgical treatment for cartilage injury or meniscus injury aims at removing debris that causes further deterioration of the joint and restoring the function of the affected joint. However, it is generally known that cartilage and meniscus tissues are difficult to self-regenerate.

On the other hand, due to the recent progress in regenerative medicine technology, cell therapy capable of repairing cartilage or meniscus has become popular. Among the above, the mesenchymal stem cell (MSC) is expected as a cell source useful for a cell therapy. It has been reported that mesenchymal stem cells can be collected from various body tissues and can be isolated from bone marrow tissue, adipose tissue, muscle tissue, synovial tissue, and bone membrane tissue (Non-Patent Document 1). In particular, it has been reported that the synovium-derived mesenchymal stem cell has a high proliferation ability and a high cartilage forming ability as compared with mesenchymal stem cells derived from various mesenchymal tissues such as bone marrow (Non-Patent Document 2). In addition, Patent Documents 1 and 2 disclose methods of medically treating articular cartilage injury and meniscus injury by using synovium-derived mesenchymal stem cells.

### Prior Art Documents

### Non-Patent Documents

Non-Patent Document 1: Na Li, et al., 2020, Stem Cell Research & Therapy. 11:381
Non-Patent Document 2: Sakaguchi, et al., 2005, Arthritis Rheum. 52:2521-9

### Patent Documents

Patent Document 1: JP5928961B
Patent Document 2: JP5656183B

### SUMMARY OF THE INVENTION

It has been revealed that karyotype abnormalities may occur in a cell culture process in stem cells including mesenchymal stem cells. In the cell transplantation for joint medical treatment, it is necessary to reduce karyotype abnormalities in order to ensure safety. In addition to ensuring safety, it is also necessary to secure a sufficient number of cells in order to achieve a therapeutic effect. Furthermore, in a case where autologous serum is used for autologous medical treatment, it is also necessary to reduce the amount of serum since there is a limit to the amount of serum that is capable of being collected.

An object to be achieved by the present invention is to provide a method of producing a cell preparation for a joint medical treatment, in which a cell proliferation ratio can be improved, an amount of serum required can be reduced, and karyotype abnormalities in a culture process can be suppressed, a method of culturing a mesenchymal stem cell, and a cell preparation for a joint medical treatment, which is produced by the above method.

As a result of diligent studies to achieve the above object, the inventors of the present invention have found that the above-described object can be achieved by culturing a mesenchymal stem cell in a culture medium containing an ascorbic acid derivative, alanyl glutamine, and pyridoxine. The present invention has been completed based on the above findings.

That is, according to the present invention, the following inventions are provided.
<1> A method of producing a cell preparation for a joint medical treatment, the method comprising:
   culturing a mesenchymal stem cell in a culture medium containing an ascorbic acid derivative, alanyl glutamine, and pyridoxine.
<2> The method according to <1>, in which the mesenchymal stem cell is a synovium-derived mesenchymal stem cell.
<3> The method according to <1> or <2>, in which the mesenchymal stem cell is of autologous origin.
<4> The method according to any one of <1> to <3>, in which the culture medium contains allogeneic serum.
<5> The method according to any one of <1> to <4>, in which the culture medium does not contain ascorbic acid.
<6> The method according to any one of <1> to <5>, in which the culture medium contains any one or more of biotin or lipoic acid.
<7> The method according to any one of <1> to <6>, in which the mesenchymal stem cell is cultured using a multilayer flask having five or more layers.
<8> The method according to any one of <1> to <7>, further comprising:
   separating a suspension of a tissue containing mesenchymal stem cells into two layers of an upper layer and a lower layer; and
   collecting the lower layer of the two layers,
   in which the mesenchymal stem cells contained in the lower layer are cultured in a culture medium containing an ascorbic acid derivative, alanyl glutamine, and pyridoxine.
<9> A cell preparation for a joint medical treatment produced by the method according to any one of <1> to <8>.
<10> The cell preparation for a joint medical treatment according to <9>, in which the cell preparation for a joint medical treatment does not contain an extracellular substrate or a scaffold.
<11> The cell preparation for a joint medical treatment according to <9> or <10>, in which the cell preparation for a joint medical treatment is a meniscus curing agent or an osteoarthritis curing agent.
<12> The cell preparation for a joint medical treatment according to any one of <9> to <11>, in which the mesenchymal stem cell is a human mesenchymal stem cell, and the number of chromosomes of the mesenchymal stem cell is 46, where the chromosomes have a karyotype of pairs of chromosomes from chromosome 1 to chromosome 22 and, XX chromosomes or XY chromosomes.
<13> A method of culturing a mesenchymal stem cell, the method comprising:
   separating a suspension of a tissue containing mesenchymal stem cells into two layers of an upper layer and a lower layer; and
   collecting the lower layer of the two layers; and
   culturing the mesenchymal stem cells contained in the lower layer in a culture medium containing an ascorbic acid derivative, alanyl glutamine, and pyridoxine.
<14> The method according to <13>, in which the mesenchymal stem cell is a synovium-derived mesenchymal stem cell.
<15> The method according to <13> or < 14>, in which the mesenchymal stem cell is of autologous origin.
<16> The method according to any one of <13> to <15>, in which the culture medium contains allogeneic serum.
<17> The method according to any one of <13> to <16>, in which the culture medium does not contain ascorbic acid.
<18> The method according to any one of <13> to <17>, in which the culture medium contains any one or more of biotin or lipoic acid.
<19> The method according to any one of <13> to <18>, in which the mesenchymal stem cell is cultured using a multilayer flask having five or more layers.
<20> A cell preparation for a joint medical treatment, comprising the mesenchymal stem cell cultured by the method according to any one of <13> to <19>.
<21> The cell preparation for a joint medical treatment according to <20>, in which the cell preparation for a joint medical treatment does not contain an extracellular substrate or a scaffold.
<22> The cell preparation for a joint medical treatment according to <20> or <21>, in which the cell preparation for a joint medical treatment is a meniscus curing agent or an osteoarthritis curing agent.
<23> The cell preparation for a joint medical treatment according to any one of <20> to <22>, in which the mesenchymal stem cell is a human mesenchymal stem cell, and the number of chromosomes of the mesenchymal stem cell is 46, where the chromosomes have a karyotype of pairs of chromosomes from chromosome 1 to chromosome 22 and, XX chromosomes or XY chromosomes.

<A> A medical treatment method for a joint, comprising:
   a step of transplanting the cell preparation for a joint medical treatment produced by the method according to an aspect of the present invention or the mesenchymal stem cell cultured by the method according to an aspect of the present invention so that a cartilage injury part or a meniscus injury part is covered by the mesenchymal stem cell; and
   a step of differentiating the mesenchymal stem cell into a cartilage cell to regenerate cartilage tissue in situ at a cartilage injury part or a meniscus injury part.

According to the present invention, in a case of producing a sufficient amount of cells from a tissue, it is possible to improve the proliferation ratio, reduce the amount of serum required, and suppress the karyotype abnormalities in a culture process.

### BRIEF DESCRIPTION OF THE DRAWINGS

Fig. 1 shows a proliferation curve of mesenchymal stem cells in a case where a type culture medium or a culture medium A is used.
Fig. 2 shows images of a karyotype analysis of mesenchymal stem cells in a case where the type culture medium or the culture medium A is used.
Fig. 3 shows images of a karyotype analysis of mesenchymal stem cells in a case where a culture medium obtained by adding bFGF to the type culture medium, or the culture medium A is used.
Fig. 4 shows images of the medial meniscus of both knee joints excised from rats administered with rat synovium-derived mesenchymal stem cells (rSMSC) or PBS.

### DESCRIPTION OF THE PREFERRED EMBODIMENTS

Hereinafter, the contents of the present invention will be described in detail. It is noted that in the present specification, "to" is used to mean that numerical values described before and after "to" are included as a lower limit value and an upper limit value, respectively.

### < 1 > Method of producing cell preparation for joint medical treatment

The present invention relates to a method of producing a cell preparation for a joint medical treatment, the method including culturing a mesenchymal stem cell in a culture medium containing an ascorbic acid derivative, alanyl glutamine, and pyridoxine. The fact that high cell proliferation can be obtained by culturing a mesenchymal stem cell in a culture medium containing the specific component described above in spite of suppressing the occurrence of the karyotype abnormalities, that is, the fact that both the suppression of the occurrence of the karyotype abnormalities and the high cell proliferation are achieved is not a fact that can be predicted from the findings in the related art, which is a completely unexpected result.

The method of producing a cell preparation for a joint medical treatment according to the present invention may include culturing a mesenchymal stem cell in a culture medium containing an ascorbic acid derivative, alanyl glutamine, and pyridoxine and producing a cell preparation for a joint medical treatment using the mesenchymal stem cell as cultured above.

### <Mesenchymal stem cell>

The mesenchymal stem cell means, in a broad sense, all mesenchymal cells such as an osteoblast, a chondroblast, a lipoblast, and a muscle cell, or stem cells capable of differentiating into a part of cells thereof, or a population of precursor cells thereof. The mesenchymal stem cells are known to be present in bone marrow, synovium, periosteum, adipose tissue, and muscle tissue. In association with the differentiation of mesenchymal stem cells into cartilage cells, it is known that the addition of bone morphogenetic protein (BMP) or transforming growth factor-β (TGF-β) to the culture solution promotes the differentiation of undifferentiated mesenchymal stem cells into cartilage cells, and the cartilage tissue can be regenerated under in vitro conditions.

The mesenchymal stem cell can be confirmed by detecting a molecule characteristic of the mesenchymal stem cell, for example, an enzyme, a receptor, a low-molecular-weight compound, or the like. Examples of the molecule characteristic of mesenchymal stem cells include, which are not limited to, CD73, CD90, CD105, and CD166, which are cell surface markers (positive markers). In addition, examples of the negative marker that are not expressed in mesenchymal stem cells include, but are not limited to, CD19, CD34, CD45, HLA-DR, CD11b, and CD14. It is noted that CD is an abbreviation for Clusters of d differentiation, and HLA-DR is an abbreviation for human leukocyte antigen -D-related. These positive markers and negative markers can be used to confirm that a cell is the mesenchymal stem cell. Although an immunological method can be used for the detection of these markers, the detection may be carried out by quantifying the mRNA amount of each molecule.

The animal species from which the mesenchymal stem cells are derived is not particularly limited, and they may be cells of rodents such as a rat, a mouse, a hamster, and a guinea pig, the Leporidae such as a rabbit, the Ungulata such as a pig, a cow, a goat, and a sheep, the Carnivora such as a dog and a cat, and primates such as a human, a monkey, a rhesus monkey, a marmoset, an orangutan, or a chimpanzee.

The mesenchymal stem cell is preferably a human mesenchymal stem cell.

The origin of the mesenchymal stem cell is not particularly limited; however, it is preferably derived from the synovium, the bone marrow, the adipose, the dental pulp, or the fetus, or derived from an induced pluripotent stem cell. The mesenchymal stem cell is more preferably a synovium-derived mesenchymal stem cell or a bone marrow-derived mesenchymal stem cell, and it is still more preferably a synovium-derived mesenchymal stem cell.

The mesenchymal stem cell may be of autologous origin or of heterologous origin; however, it is preferably of autologous origin.

The mesenchymal stem cell may be a genetically modified cell or may be a non-genetically modified cell; however, it is preferably a non-genetically modified cell.

The mesenchymal stem cells can be collected from the above-described tissues by a conventional method. Preferably, the mesenchymal stem cell can be collected by separating a suspension of a tissue containing mesenchymal stem cells into two layers of an upper layer and a lower layer, and then collecting the lower layer of the two layers. Separating a suspension of a tissue containing mesenchymal stem cells into two layers of an upper layer and a lower layer can be carried out, for example, by centrifugation.

As an example, a method of collecting synovium-derived mesenchymal stem cells from the synovial tissue will be described.

The synovial tissue can be collected from the unloaded portion of the joint under anesthesia. The amount of the synovial tissue to be collected can be determined in consideration of the type of donor or the required amount of synovium-derived mesenchymal stem cells. For example, it is possible to obtain synovium-derived mesenchymal stem cells from the synovial tissue of 0.1 g to 10 g, preferably 0.1 g to 2.0 g, more preferably 0.1 g to 1.5 g, and still more preferably 0.1 g to 1.0 g. The collected synovial tissue is shredded with scissors or the like as necessary, and then subjected to an enzyme treatment. The enzyme is not particularly limited as long as it is an enzyme including a protease. However, it is preferably a mixed enzyme including one or more kinds of collagenases and one or more kinds of neutral proteases. A particularly preferred enzyme is a liberase. As the liberase, it is possible to use, for example, Liberase MNP-S (manufactured by F. Hoffmann-La Roche, Ltd.), which is an enzyme including a collagenase class I, a collagenase class II, and a neutral protease (thermolysin). The enzyme concentration in the enzyme treatment is preferably 0.01 mg/ml to 10 mg/ml, more preferably 0.1 mg/ml to 10 mg/ml, still more preferably 0.5 mg/ml to 10 mg/ml, ever still more preferably 0.5 mg/ml to 5.0 mg/ml, particularly preferably 0.5 mg/ml to 2.0 mg/ml, and most preferably 0.7 mg/ml to 2.0 mg/ml. The mass ratio of the synovial tissue to the enzyme is preferably 1,000:1 to 10:1, more preferably 500:1 to 20:1, and still more preferably 200:1 to 40:1.

The enzymatic reaction can be carried out at a temperature of preferably 15°C to 40°C and more preferably 20°C to 40°C. It suffices that the reaction time is 30 minutes or more, where the reaction time is preferably 2 hours or more, more preferably 2 hours and 30 minutes or more, and still more preferably 3 hours or more. The upper limit of the reaction time is not particularly limited; however, it is generally 4 hours or less. The enzyme-treated mixture contains synovium-derived mesenchymal stem cells. The enzyme-treated mixture is transferred to a centrifuge tube through a cell strainer and centrifuged, whereby synovium-derived mesenchymal stem cells can be recovered.

### <Culture medium>

The culture medium that is used in the present invention preferably contains essential amino acids. That is, the culture medium preferably contains histidine, isoleucine, leucine, lysine, methionine, phenylalanine, threonine, tryptophan, and valine. The concentration of the essential amino acids in the culture medium is not particularly limited; however, the concentration of each of the essential amino acids is preferably 0.003 mmol/L or more, more preferably 0.005 mmol/L or more, and still more preferably 0.01 mmol/L or more. The upper limit value thereof is generally 5 mmol/L or less.

The total concentration of the essential amino acids is preferably 0.5 mmol/L or more, more preferably 1 mmol/L or more, and still more preferably 1.5 mmol/L or more. The upper limit value thereof is generally 15 mmol/L or less.

The culture medium preferably contains non-essential amino acids and the like. Here, the non-essential amino acids and the like are defined to mean non-essential amino acids and glutamines. Examples of the non-essential amino acids include one or more selected from the group consisting of glycine, alanine, arginine, asparagine, aspartic acid, cysteine, cystine, glutamines, glutamic acid, proline, serine, and tyrosine.

In a case where glycine, alanine, serine, proline, asparagine, aspartic acid, glutamines, or/and glutamic acid are included as non-essential amino acids and the like, each of these is 0.005 mmol/L (5 pmol/L) or more, where it is preferably 0.01 mmol/L or more and more preferably 0.05 mmol/L or more. The upper limit value thereof is generally 3 mmol/L or less. Preferred examples of the glutamines include alanyl glutamine. However, the culture medium that is used in the present invention contains alanyl glutamine as an essential component.

The total concentration of the non-essential amino acids other than alanyl glutamine is preferably 0.5 mmol/L or more, more preferably 1.5 mmol/L or more, and still more preferably 2.5 mmol/L or more. The upper limit value thereof is generally 30 mmol/L or less.

The total concentration of alanyl glutamine is preferably 0.5 mmol/L or more, more preferably 1.0 mmol/L or more, and still more preferably 1.5 mmol/L or more. The upper limit value thereof is generally 10 mmol/L or less.

The culture medium preferably does not contain glutamine. Here, "does not contain" means being substantially not contained in association with the effect of the present invention and thus does not mean to exclude those that are unavoidably mixed. Examples of being substantially not contained are generally less than 5 × 10⁻⁵ mmol/L and preferably 0 mmol/L.

The detection and measurement methods for the amino acid may be carried out according to known methods. However, they can be carried out, for example, according to a quantification of an amino acid by high-speed liquid chromatography (HPLC) and an amino acid analysis method by the ninhydrin method (for example, see Clinical Chemistry (1997), Vol. 43, No. 8. p1421-1428).

The amino acid described in the present specification may be any of an L-form, a D-form, or a DL-form. In addition, the amino acid may form not only a liberated form but a salt. Examples of the salt form include an acid addition salt and a salt with a base. Examples of the acid include inorganic acids such as hydrogen chloride, hydrogen bromide, sulfuric acid, and phosphoric acid; and organic acids such as acetic acid, lactic acid, citric acid, tartaric acid, maleic acid, fumaric acid, and monomethylsulfuric acid. Examples of the base that forms such a salt include inorganic bases such as a hydroxide or carbonate of a metal (for example, sodium, potassium, or calcium), and ammonia; and organic bases such as ethylenediamine, propylenediamine, ethanolamine, a monoalkylethanolamine, a dialkylethanolamine, diethanolamine, and triethanolamine. The salt may be a hydrate (a hydrous salt).

The culture medium contains pyridoxine.

The culture medium may contain biotin.

The culture medium may further contain at least one kind of other vitamins, in addition to pyridoxine and biotin. Examples of the other vitamins include vitamin B12, choline chloride, calcium pantothenate, folic acid, niacinamide, pyridoxals (excluding pyridoxine), riboflavin, thiamine hydrochloride, and i-inositol. Examples of the pyridoxals include pyridoxal.

The above-described pyridoxine, biotin, and at least one kind of other vitamins may form not only a liberated form but a salt. Examples of the salt form include an acid addition salt and a salt with a base. Specific examples thereof include those described above regarding the amino acid.

The content concentration of each of pyridoxine, biotin, and at least one kind of other vitamins in the culture medium is preferably 0.00005 mmol/L or more, more preferably 0.0001 mmol/L or more, and still more preferably 0.0002 mmol/L or more. The upper limit value thereof is generally 1 mmol/L or less.

The content concentration of the vitamins in the culture medium is, in total, preferably 0.001 mmol/L or more, more preferably 0.005 mmol/L or more, and still more preferably 0.01 mmol/L or more. The upper limit value thereof is generally 2 mmol/L or less.

It is preferable that the culture medium does not contain pyridoxal or pyridoxals (excluding pyridoxine). Here, "does not contain" means being substantially not contained in association with the effect of the present invention and thus does not mean to exclude those that are unavoidably mixed. In a case of pyridoxal, examples of being substantially not contained are generally less than 5 × 10⁻⁵ mmol/L and preferably 0 mmol/L.

The culture medium preferably contains at least one inorganic salt. The inorganic salt is preferably one or more selected from the group consisting of calcium chloride, magnesium sulfate, potassium chloride, sodium hydrogen carbonate, sodium chloride, and sodium dihydrogen phosphate. The concentration of the inorganic salt is not particularly limited; however, it is preferably, in total, 10 mmol/L or more, more preferably 50 mmol/L or more, and still more preferably 80 mmol/L or more. The upper limit value thereof is generally 1,000 mmol/L or less.

The culture medium preferably contains at least one kind of sugars or a pyruvate. Examples of the sugars include D-glucose. Examples of the pyruvate include sodium pyruvate. The concentrations of the sugars and the pyruvate are preferably, in total, 0.1 mmol/L or more, more preferably 0.3 mmol/L or more, and still more preferably 1 mmol/L or more. The upper limit value thereof is generally 50 mmol/L or less.

The culture medium preferably contains lipoic acid. The concentration of lipoic acid is preferably 5 × 10⁻⁵ mmol/L or more, more preferably 0.0001 mmol/L or more, and still more preferably 0.0005 mmol/L or more. The upper limit value thereof is generally 0.005 mmol/L or less.

The culture medium that is used in the present invention contains an ascorbic acid derivative instead of the ascorbic acid. Examples of the ascorbic acid derivative include ascorbic acid-2-phosphate, an ascorbic acid-2-phosphate trisodium salt, an ascorbic acid-2-phosphate magnesium salt, and ascorbic acid-2-glycoside, where one kind may be selected from a group of these derivatives and used, or two or more kinds thereof may be combined. An L-ascorbic acid-2-phosphate trisodium salt is preferably used.

The culture medium that is used in the present invention preferably does not contain ascorbic acid.

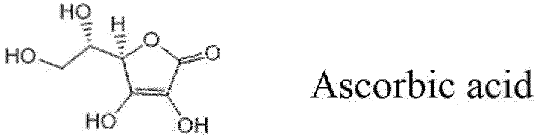

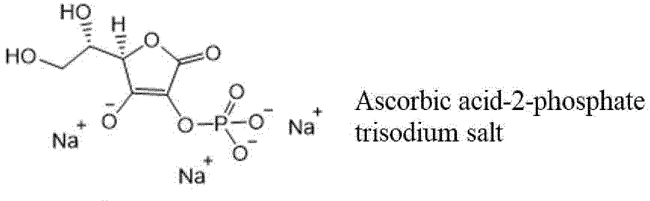

In the present invention, it is important to contain an ascorbic acid derivative instead of ascorbic acid. It is conceived that this is because the stability as a culture medium is improved by employing an ascorbic acid derivative instead of the unstable ascorbic acid.

In the culture medium composition according to the embodiment of the present invention, the concentration of the ascorbic acid derivative is, in total, preferably 0.03 mmol/L or more, more preferably 0.1 mmol/L or more, and still more preferably 0.14 mmol/L or more. The upper limit value thereof is preferably 5.0 mmol/L or less, more preferably 1.0 mmol/L or less, and still more preferably 0.57 mmol/L or less.

The culture medium preferably does not contain linoleic acid. Further, the culture medium preferably does not contain a nucleic acid.

Here, "does not contain" means being substantially not contained in association with the effect of the present invention and thus does not mean to exclude those that are unavoidably mixed. In a case of ascorbic acid, examples of being substantially not contained are generally less than 5 × 10⁻⁵ mmol/L and preferably 0 mmol/L. In a case of linoleic acid, it is generally less than 0.0015 mmol/L, preferably 0.001 mmol/L or less, more preferably 0.0005 mmol/L or less, still more preferably 0.0003 mmol/L or less, particularly preferably 0.00015 mmol/L or less, and most preferably 0 mmol/L. In a case of a nucleic acid, it is generally less than 5 × 10⁻⁵ mmol/L and preferably 0 mmol/L.

It is known that mesenchymal stem cells are differentiated into cartilage cells by culturing them in a cartilage forming culture medium to which transforming proliferation factor β3 (TGF-β3), dexamethasone, and bone morphogenetic protein 2 (BMP-2) have been added, whereby the cartilage tissue can be prepared in vitro. Accordingly, in order for a mesenchymal stem cell not to differentiate into a cartilage cell, it is preferable that the culture medium does not contain TGF-β3, dexamethasone, and BMP-2.

Here, "does not contain" means being substantially not contained and thus does not mean excluding those that are unavoidably mixed. In a case of TGF-β3, examples of being substantially not contained are generally less than 0.1 ng/mL and preferably 0 ng/mL. In a case of dexamethasone, it is generally less than 1.0 nmol/L and preferably 0 nmol/L. In addition, in a case of BMP-2, it is generally less than 0.1 ng/mL and preferably 0 ng/mL.

It is preferable that the culture medium does not contain insulin, transferrin, selenic acid, or bovine serum albumin (BSA).

Here, "does not contain" means being substantially not contained and thus does not mean excluding those that are unavoidably mixed. In a case of insulin, examples of being substantially not contained are generally less than 10 ng/mL and preferably 0 ng/mL.

In a case of transferrin, it is generally less than 10 ng/mL preferably 0 ng/mL. In a case of selenic acid, it is generally less than 0.01 ng/mL and preferably 0 ng/mL. In a case of BSA, it is generally less than 10 µg/mL and preferably 0 µg/mL.

The culture medium may contain phenol red. The concentration of the phenol red is preferably 0.001 mmol/L or more, more preferably 0.005 mmol/L or more, and still more preferably 0.01 mmol/L or more. The upper limit value thereof is generally 0.2 mmol/L or less.

The culture medium may be a culture medium containing serum or may be a culture medium not containing serum. The lower limit of the amount of serum in the culture medium containing the serum is preferably 2% by volume or more, more preferably 5% by volume or more, and still more preferably 10% by volume or more. The upper limit value thereof is generally 20% by volume or less.

Examples of the serum include serum derived from an animal, where human serum is preferable.

The serum may be allogeneic serum or heterologous serum, where it is preferably allogeneic serum. That is, in a case of producing a mesenchymal stem cell from a human tissue for the intended purpose of administration to a human, a culture medium containing a human serum may be used. In a case where allogeneic serum is used, the serum may be an autologous serum or may be an allogeneic serum; however, it is preferably an autologous serum.

The culture medium may further contain an antibiotic. Examples of the antibiotic include streptomycin, gentamycin (gentamycin sulfate or the like), penicillin, and amphotericin B. The concentration of the antibiotics, as a total concentration thereof, is preferably 0.1 mg/L or more, more preferably 0.5 mg/L or more, and still more preferably 10.0 mg/L or more. The upper limit value thereof is generally 1,000 mg/L or less.

The culture medium can contain, as necessary, a known additive in addition to the above-described components. Examples of the additive include polyamines (for example, putrescine and the like), a reducing agent (for example, 2-mercaptoethanol or the like), and a buffering agent (for example, 4-(2-hydroxyethyl)-1-piperazineethanesulfonic acid (HEPES)).

### <Culturing method>

The culture vessel that is used for culturing mesenchymal stem cells is not particularly limited as long as it enables the culture of mesenchymal stem cells. However, examples thereof include a flask, a tissue culture flask, a dish, a petri dish, a tissue culture dish, a multi-dish, a micro-plate, a micro-well plate, a multi-plate, a multi-well plate, a micro-slide, a chamber slide, a chalet, a tube, a tray, a culture bag, and a roller bottle. In the present invention, preferably, mesenchymal stem cells are cultured using a multilayer flask having 5 or more layers (for example, 5 to 10 layers). As an example of the multilayer flask having 5 or more layers, a 10-cell stack (CellSTACK-10 Chamber made of polystyrene, manufactured by Corning Inc.) can be used. As the flask, a flask equipped with a vent cap can also be used. In a case of using a flask equipped with a vent cap and carrying out culturing while forcibly aerating gas from the vent cap, the proliferation rate of mesenchymal stem cells can be significantly improved.

The culture vessel may be cell-adhesive or cell-non-adhesive, and it is appropriately selected depending on the intended purpose. The cell-adhesive culture vessel may be coated with any cell-supporting substrate such as an extracellular matrix (ECM), for the intended purpose of improving the adhesiveness of the surface of the culture vessel to cells. The cell-supporting substrate can be any substance for the intended purpose of adhering mesenchymal stem cells, and examples thereof include Matrigel using ECM, collagen, gelatin, poly-L-lysine, poly-D-lysine, laminin, and fibronectin.

The culture conditions can be appropriately set. For example, the culture temperature is not particularly limited; however, it may be about 30°C to 40°C and preferably about 37°C. The CO₂ concentration may be about 1% to 10% and preferably about 2% to 5%. The oxygen concentration may be 1% to 20% and preferably 1% to 10%.

It is preferable to adjust the culture period in order to proliferate mesenchymal stem cells in an undifferentiated state and in a state of having a good in situ cartilage forming ability. In addition, it is necessary to consider the need to prepare a sufficient number of undifferentiated mesenchymal stem cells in order to cover the cartilage injury part and regenerate the affected part. Accordingly, the culture period of the mesenchymal stem cells is preferably 5 days or more, 7 days or more, and 10 days or more, and it may be 10 to 100 days, 10 to 90 days, 10 to 80 days, 10 to 70 days, 10 to 60 days, 10 to 50 days, 10 to 40 days, 10 to 30 days, 10 to 28 days, 10 to 21 days, or 10 to 14 days. In a case of using a synovium-derived mesenchymal stem cell as the mesenchymal stem cell, It is preferable to adjust the culture period in order to proliferate a synovium-derived mesenchymal stem cell in an undifferentiated state and in a state of having a good in situ cartilage forming ability. In addition, it is necessary to consider the need to prepare a sufficient number of undifferentiated synovium-derived mesenchymal stem cells in order to cover the cartilage injury part and regenerate the affected part. Accordingly, the culture period of the synovium-derived mesenchymal stem cells is preferably 5 days or more, 7 days or more, and 10 days or more, and it may be 10 to 100 days, 10 to 90 days, 10 to 80 days, 10 to 70 days, 10 to 60 days, 10 to 50 days, 10 to 40 days, 10 to 30 days, 10 to 28 days, 10 to 21 days, or 10 to 14 days.

It is also known that in the mesenchymal stem cells, the in situ cartilage forming ability decreases in inverse proportion to the number of passages of the mesenchymal stem cells in vitro. Accordingly, in order to prepare undifferentiated mesenchymal stem cells, the number of passages is preferably 10 or less and more preferably 5 or less, and it is still more preferable that the mesenchymal stem cells are produced in the primary or first passage.

It is also known that in the synovium-derived mesenchymal stem cells, the in situ cartilage forming ability decreases in inverse proportion to the number of passages of the synovium-derived mesenchymal stem cells in vitro. Accordingly, in order to prepare undifferentiated synovium-derived mesenchymal stem cells, the number of passages is preferably 10 or less and more preferably 5 or less.

### <2> Method of culturing mesenchymal stem cell

The present invention relates to a method of culturing a mesenchymal stem cell, the method including:
separating a suspension of a tissue containing mesenchymal stem cells into two layers of an upper layer and a lower layer; and
collecting the lower layer of the two layers; and
culturing the mesenchymal stem cells contained in the lower layer in a culture medium containing an ascorbic acid-2-phosphate trisodium salt, alanyl glutamine, and pyridoxine.

Specific examples and preferred embodiments of the mesenchymal stem cell, the culture medium, and the culturing method in the above-described method of culturing a mesenchymal stem cell according to the present invention are as described above in the present specification.

### <3> Cell preparation for joint medical treatment

According to the present invention, there is provided a cell preparation for a joint medical treatment produced according to the method of producing a cell preparation for a joint medical treatment according to the present invention. According to the present invention, there is further provided a cell preparation for a joint medical treatment, which contains the mesenchymal stem cells cultured by the method of culturing a mesenchymal stem cell according to the present invention.

According to the present invention, it is possible to produce a cell preparation for a joint medical treatment, which contains the mesenchymal stem cell obtained by the method according to the embodiment of the present invention as an active ingredient.

The cell preparation for a joint medical treatment preferably does not contain an extracellular substrate or a scaffold.

Examples of the extracellular substrate or scaffold referred to here include collagen, hyaluronic acid, alginic acid, polylactic acid, and polyglycolic acid.

The mesenchymal stem cell to be used as an active ingredient in the cell preparation for a joint medical treatment is preferably a cell that does not have karyotype abnormalities. In a case of a human mesenchymal stem cell, examples of the cell that does not have karyotype abnormalities include a cell in which the number of chromosomes of the mesenchymal stem cell is 46, where the chromosomes have a karyotype of pairs of chromosomes from chromosome 1 to chromosome 22 and, XX chromosomes or XY chromosomes.

Regarding the mesenchymal stem cells that are used as the active ingredient in the cell preparation for a joint medical treatment, the ratio of normal cells that do not have the karyotype abnormalities as described above is preferably higher than 90%, more preferably 91% or higher, still preferably 93% or more, even more preferably 95% or more, even still more preferably 98% or more, particularly preferably 99% or more, and most preferably 100%.

Examples of the joint medical treatment include a medical treatment for a disease associated with injury, damage, or inflammation of the joint, and examples of the medical treatment include a medical treatment for a joint disease resulting from degeneration and/or inflammation of the connective tissue such as cartilage, or a non-inflammatory joint disease. Examples of the joint medical treatment include a medical treatment for a disease selected from the group consisting of meniscus injury, traumatic cartilage injury, osteochondritis dissecans, aseptic osteonecrosis, osteoarthritis, rheumatoid arthritis (for example, chronic rheumatoid arthritis), gout, reactive arthritis, psoriatic arthritis, juvenile arthritis, inflammatory arthritis, and articular cartilage defect, which are not limited to these diseases. The osteoarthritis may be gonarthrosis in which the joint part is the knee, where the joint part may be the elbow joint, the finger joint, the hip joint, the shoulder joint, the ankle joint, or the cervical spine, or may be a combination of a plurality of joint parts. The cell preparation for a joint medical treatment according to the present invention is preferably a meniscus curing agent or an osteoarthritis curing agent.

In a case of producing a cell preparation for a joint medical treatment, the mesenchymal stem cells may be made into a pharmaceutical preparation in a form suitable for administration to an individual, by being mixed with a pharmaceutically acceptable carrier according to a conventional method. Examples of the carrier include physiological saline and distilled water for injection, which has been made to be isotonic by adding glucose and other auxiliary agents (for example, D-sorbitol, D-mannitol, sodium chloride, and the like). Further, a buffering agent (for example, a phosphate buffer solution or a sodium acetate buffer solution), a soothing agent (for example, benzalkonium chloride, procaine hydrochloride, or the like), a stabilizer (for example, human serum albumin, polyethylene glycol, or the like), a storage agent, an antioxidant, and the like may be blended.

### [Medical treatment method for joint]

The present invention further relates to a medical treatment method for a joint. More specifically, the present invention relates to a medical treatment method for a disease selected from the group consisting of meniscus injury, traumatic cartilage injury, osteochondritis dissecans, aseptic osteonecrosis, osteoarthritis (for example, gonarthrosis in which the joint part is the knee, where the joint part is the elbow joint, the finger joint, the hip joint, the shoulder joint, the ankle joint, the cervical spine, and a combination of a plurality of joint parts), rheumatoid arthritis (for example, chronic rheumatoid arthritis), gout, reactive arthritis, psoriatic arthritis, juvenile arthritis, inflammatory arthritis, and articular cartilage defect.

The medical treatment method for a joint according to the embodiment of the present invention includes;
a step of transplanting the joint curing agent produced by the method according to the embodiment of the present invention so that a cartilage injury part or a meniscus injury part is covered by the mesenchymal stem cell, and
a step of differentiating the mesenchymal stem cell into a cartilage cell to regenerate cartilage tissue in situ at a cartilage injury part or a meniscus injury part.

In a case where the joint curing agent according to the embodiment of the present invention is transplanted into a patient, it is preferable to apply, per cartilage injury part or meniscus injury part, mesenchymal stem cells of 1 × 10⁶ to 1.0 × 10¹¹ cells, 2 × 10⁶ to 1.0 × 10¹¹ cells, 5 × 10⁶ to 1.0 × 10¹¹ cells, 1 × 10⁷ to 1.0 × 10¹¹ cells, 2.0 × 10⁷ to 1.0 × 10¹¹ cells, 2.5 × 10⁷ to 1.0 × 10¹¹ cells, 3.0 × 10⁷ to 1.0 × 10¹¹ cells, 4.0 × 10⁷ to 1.0 × 10¹¹ cells, 1 × 10⁶ to 1.0 × 10¹⁰ cells, 2.5 × 10⁷ to 1.0 × 10¹⁰ cells, 1 × 10⁶ to 1.0 × 10⁹ cells, 2.5 × 10⁷ to 1.0 × 10⁹ cells, 1 × 10⁶ to 1.0 × 10⁸ cells, 2.5 × 10⁷ to 1.0 × 10⁸ cells, or 2.0 × 10⁷ to 1.0 × 10⁸ cells, and more preferably mesenchymal stem cells of 2.0 × 10⁷ to 1.0 × 10⁸ cells, in order to efficiently treat medically the cartilage injury part or the meniscus injury part.

By transplanting mesenchymal stem cells into the cartilage injury part or the meniscus injury part, the cartilage injury part or the meniscus injury part is covered with the mesenchymal stem cells. The transplantation of the mesenchymal stem cell can be carried out by open surgery or by arthroscopic surgery. In order to minimize the invasion, it is preferable to arthroscopically transplant the mesenchymal stem cell.

The cartilage injury part or the meniscus injury part may be covered with a suspension of mesenchymal stem cells or may be covered with a cell sheet of mesenchymal stem cells. The mesenchymal stem cell has a high ability to adhere to the cartilage injury part or the meniscus injury part.

In a case of a medical treatment for cartilage injury, the minimally invasive method according to the embodiment of the present invention is characterized in that the cartilage injury part is covered with mesenchymal stem cells, and it includes the following steps:
holding the body position so that the cartilage injury part faces upward;
placing a cell sheet of mesenchymal stem cells, a suspension of mesenchymal stem cells, or a gel-like substance containing mesenchymal stem cells on the surface of the cartilage injury part; and
holding the body position for a specific period of time to allow the mesenchymal stem cells to adhere to the surface of the cartilage injury part.

In a case of a medical treatment for a meniscus injury, the minimally invasive method according to the embodiment of the present invention is characterized in that the meniscus injury part is covered with mesenchymal stem cells, and it includes the following steps:
holding the body position so that the meniscus injury part faces downward;
injecting the suspension of mesenchymal stem cells into a knee joint; and
holding the body position for a specific period of time to allow the mesenchymal stem cells to adhere to the meniscus injury part.

In order to reliably allow the mesenchymal stem cells to adhere to the surface of the cartilage injury part or the meniscus injury part, the transplanted mesenchymal stem cells on the surface of the cartilage injury part or meniscus injury part is held for at least 10 minutes and preferably 15 minutes. In order to achieve this, the body position is held for at least 10 minutes and preferably 15 minutes in order to achieve that the cartilage injury part or the meniscus injury part faces upward and that the mesenchymal stem cells are held in the cartilage injury part or the meniscus injury part, which has faced upward.

In order to further strengthen the adhesion of the mesenchymal stem cells to the cartilage injury part or the meniscus injury part, the cartilage injury part or the meniscus injury part associated with the mesenchymal stem cells can be covered with the periosteum. The operation is completed after holding the mesenchymal stem cells on the surface of the cartilage injury part or the surface of the meniscus injury part for at least 10 minutes.

In the present invention, the transplanted mesenchymal stem cells are differentiated into cartilage cells at the cartilage injury part or the meniscus injury part and the cartilage tissue is regenerated in situ at the cartilage injury part or the meniscus injury part.

During the in situ cartilage formation process of the mesenchymal stem cells, the cartilage tissue is regenerated depending on the local microenvironment (the nutrient supply, the cytokine environment, and the like), and thus no external operation is required. As a result of the in situ cartilage formation of synovium-derived mesenchymal stem cells, the cartilage tissue is regenerated at the cartilage injury part or the meniscus injury part to repair the injury. Then, in a case of cartilage injury, a bone region, a boundary between the cartilage and the bone, a central part of the cartilage, a surface region, and a region adjacent to the original cartilage are formed as the original cartilage tissue, or in a case of the meniscus injury, meniscus cartilage is formed.

The present invention will be further specifically described with reference to Examples; however, the present invention is not limited by Examples.

### Examples

### [Experiment A]

### <Materials and methods>

### (1) Cell

A mesenchymal stem cell (MSC) isolated from human bone marrow tissue (Whole Bone Marrow, Fresh, 10 mL, model number: ALL-ABM001, lot: B009, B012) purchased from AllCells, LLC was used.

### (2) Basal medium

### Type basal medium:

MEM alpha no nucleosides (model number: 12561), manufactured by Thermo Fisher Scientific, Inc.

### Basal medium A:

A culture medium obtained by replacing ascorbic acid, glutamine, and pyridoxal in the type culture medium with an ascorbic acid-2-phosphate trisodium salt, alanyl glutamine, and pyridoxine, respectively.

Table 1 shows the culture medium compositions of the type basal medium and the basal medium A.

**[Table 1]**

| Component | | Type basal medium | | Basal medium A | |
|---|---|---|---|---|---|
| | Details | Model number: 12561 (Thermo) | | APN, Ala-Gln, pyridoxin, ph+ | |
| | Molecular weight | Concentration (mg/L) | mmol/L | Concentration (mg/L) | mmol/L |
| Amino acid | | | | | |
| Glycine | 75 | 50 | 0.667 | 50 | 0.667 |
| L-alanine | 89 | 25 | 0.281 | 25 | 0.281 |
| L-arginine hydrochloride | 211 | 127 | 0.602 | 127 | 0.602 |
| L-asparagine hydrate | 150 | 50 | 0.333 | 50 | 0.333 |
| L-aspartic acid | 133 | 30 | 0.226 | 30 | 0.226 |
| L-cystine dihydrochloride | 313 | 31 | 0.099 | 31 | 0.099 |
| L-cvsteine hydrochloride hydrate | 176 | 100 | 0.568 | 100 | 0.568 |
| L-glutamic acid | 147 | 75 | 0.510 | 75 | 0.510 |
| L-glutamine | 146 | 292 | 2 | 0 | 0 |
| L-alanyl glutamine | 217 | 0 | 0 | 434 | 2 |
| L-histidine | 155 | 0 | 0 | 31 | 0.2 |
| L-histidine hydrochloride hydrate | 210 | 42 | 0.2 | 0 | 0 |
| L-isoleucine | 131 | 52.4 | 0.4 | 52.4 | 0.4 |
| L-leucine | 131 | 52 | 0.397 | 52 | 0.397 |
| L-lysine hydrochloride | 183 | 73 | 0.399 | 73 | 0.399 |
| L-methionine | 149 | 15 | 0.101 | 15 | 0.101 |
| L-phenylalanine | 165 | 32 | 0.194 | 32 | 0.194 |
| L-proline | 115 | 40 | 0.348 | 40 | 0.348 |
| L-serine | 105 | 25 | 0.238 | 25 | 0.238 |
| L-threonine | 119 | 48 | 0.403 | 48 | 0.403 |
| L-tryptophan | 204 | 10 | 0.049 | 10 | 0.049 |
| L-tyrosine disodium hydrate | 261 | 52 | 0.198 | 52 | 0.198 |
| L-valine | 117 | 46 | 0.393 | 46 | 0.393 |

| Vitamin | | | | | |
|---|---|---|---|---|---|
| Ascorbic acid | 176 | 50 | 0.284 | 0 | 0 |
| L-ascorbic acid-2-phosphate trisodium salt | 322 | 0 | 0 | 91.5 | 0.284 |
| Biotin | 244 | 0.1 | 4.10E-04 | 0.1 | 4.10E-04 |
| Choline chloride | 140 | 1 | 0.007 | 1 | 0.007 |
| Calcium D-pantothenate | 477 | 1 | 0.002 | 1 | 0.002 |
| Folic acid | 441 | 1 | 0.002 | 1 | 0.002 |
| Niacinamide | 122 | 1 | 0.008 | 1 | 0.008 |
| Pyridoxal hydrochloride | 204 | 1.0 | 0.005 | 0 | 0 |
| Pyridoxin hydrochloride | 206 | 0 | 0 | 1 | 0.005 |
| Riboflavin | 376 | 0.1 | 2.66E-04 | 0.1 | 2.66E-04 |
| Thiamine hydrochloride | 337 | 1 | 0.003 | 1 | 0.003 |
| Vitamin B12 | 1355 | 1.36 | 0.001 | 1.36 | 0.001 |
| i-inositol | 180 | 2 | 0.011 | 2 | 0.011 |

| Inorganic salt | | | | | |
|---|---|---|---|---|---|
| Calcium chloride (CaCl2) (anhydrous) | 111 | 200 | 1.802 | 200 | 1.802 |
| Magnesium sulfate (MgSO4) (anhydrous) | 120 | 97.67 | 0.814 | 97.67 | 0.814 |
| Potassium chloride (KCl) | 75 | 400 | 5.333 | 400 | 5.333 |
| Sodium hydrogen carbonate (NaHCO3) | 84 | 2200 | 26.190 | 2200 | 26.190 |
| Sodium chloride (NaCl) | 58 | 6800 | 117.241 | 6800 | 117.241 |
| Sodium dihydrogen phosphate-base anhydride (NaH2PO4) | 120 | 0 | 0 | 121.7 | 1.014 |
| Sodium dihydrogen phosphate-base (NaH2PO4-H2O) | 138 | 140 | 1.014 | 0 | 0 |

| Other components | | | | | |
|---|---|---|---|---|---|
| D-glucose (dextrose) | 180 | 1000 | 5.556 | 1000 | 5.556 |
| Phenol red | 376.4 | 10 | 0.027 | 10 | 0.027 |
| Lipoic acid | 206 | 0.2 | 9.71E-04 | 0.2 | 9.71E-04 |
| Sodium pyruvate | 110 | 110 | 1 | 110 | 1 |

### (3) Culture of mesenchymal stem cell

For the culture of mesenchymal stem cells, 20 µg/mL of gentamycin sulfate ("Gentacin Injection 60" manufactured by Takata Seiyaku Co., Ltd.) as an antibiotic and bovine fetal serum (model number: 12007C, manufactured by SAFC Biosciences, or model number: FBS-04, manufactured by Selborne Biological Services) were added to each of the basal media described in (2) so that the final concentration of the bovine fetal serum was 15% (v/v), thereby obtaining culture media which were subsequently used. Hereinafter, these will be respectively referred to as a type culture medium and a culture medium A. In addition, bFGF (which was prepared to be 10 µg/mL by dissolving FIBLAST Spray 250 (KAKEN PHARMACEUTICAL CO., LTD.) in water for injection) was added to the above-described type culture medium so that the final concentration was 10 ng/mL, thereby obtaining culture media which was subsequently used in order to improve the proliferation ability. Hereinafter, this will be referred to as a type culture medium + bFGF. It is noted that bFGF is an abbreviation for basic fibroblast growth factor.

For the isolation of mesenchymal stem cells, a tissue suspension of a purchased human bone marrow tissue (lots: B009 and B012) is centrifuged at 1,000 rpm for 10 minutes to be separated into two layers. A lower layer thereof is collected and used. The type culture medium, the culture medium A, or the type culture medium + bFGF was added to the lower layer from which the blood plasma had been removed so that the amount thereof was 10 times the amount before the blood plasma was removed, followed by seeding in a flask to 0.20 mL/cm², and culturing was carried out in an incubator at 37°C and in a 5% CO₂ atmosphere. The culture medium was exchanged every 3 or 4 days, and the cells were cultured for 12 to 13 days after seeding and then detached with a 0.05% trypsin-EDTA solution (hereinafter, referred to as trypsin, Life Technologies, model number: 25300). After the detachment, trypsin was neutralized with an equal amount of the culture medium, followed by transferring to a tube prepared separately. The culture flask was further washed with an equal amount of the culture medium, which was subsequently added to the tube, and the remaining cells were recovered. The tube was centrifuged at 200 × g for 5 minutes, and the supernatant was removed. An appropriate amount of the culture medium was added to the remaining pellet, and cell counting was carried out using a hemocytometer. The seedling was carried out in a flask so that the final concentration was 0.20 to 0.50 × 10⁴ cells/cm², and the culturing was carried out in an incubator at 37°C and in a 5% CO₂ atmosphere. Culturing was carried out for 3 days or 4 days, and when 60% or more of the bottom surface of the flask was filled with cells, subculture was repeated in the same operation as described above. At the end of the 9th passage in a case of the B009 or at the end of the 4th passage in a case of the B012, the cells were centrifuged and adjusted to 100 × 10⁴ cells/cm² with a cryopreservation solution mixed with the type culture medium so that the final concentration of dimethyl sulfoxide (Sigma-Aldrich Co., LLC, model number: D2650) was 10% (v/v), and a cell stock for evaluation for karyotype analysis was prepared.

### (4) Karyotype analysis

Each prepared cell stock was thawed in a water bath at 37°C, followed by centrifugation at 200 × g for 5 minutes, the cryopreservation solution was removed, each culture medium was added thereto, the seedling was carried out in a flask so that the final concentration was 0.35 to 0.50 × 10⁴ cells/cm², and the culturing was carried out in an incubator at 37°C and in a 5% CO₂ atmosphere for 3 or 4 days. When 60% or more of the bottom surface of the flask was filled with cells, the cells were detached with trypsin, the culture medium for recovered cells was replaced with each culture medium by the method described above, the seedling was carried out in a flask so that the final concentration was 0.25 to 0.50 × 10⁴ cells/cm², and the culturing was carried out in an incubator at 37°C and in a 5% CO₂ atmosphere for 3 or 4 days.

A demecolcine solution (Fujifilm Wako Pure Chemical Corporation, model number: 045-18761) of 1/100 times the amount of the culture medium was added to each culture flask, which was subsequently allowed to stand in an incubator at 37°C and in a 5% CO₂ atmosphere for 3 to 4 hours. After the completion of the reaction, each of the cells was detached with trypsin. Then, Carnoy fixation was carried out according to a method recommended by Trans Chromosomics, Inc., and a Q-band analysis was requested.

### <Result>

### (1) Comparison of culturing and karyotype analysis of MSC using type culture medium or culture medium A

Each of the frozen stocks (P10) of MSC that had been subjected to 9 passages in the type culture medium of lot: B009 and the culture medium A were thawed, subculturing was carried out twice in each culture medium, and the karyotype analysis of the cells of P12 (the cells subjected to 11 passages) was carried out.

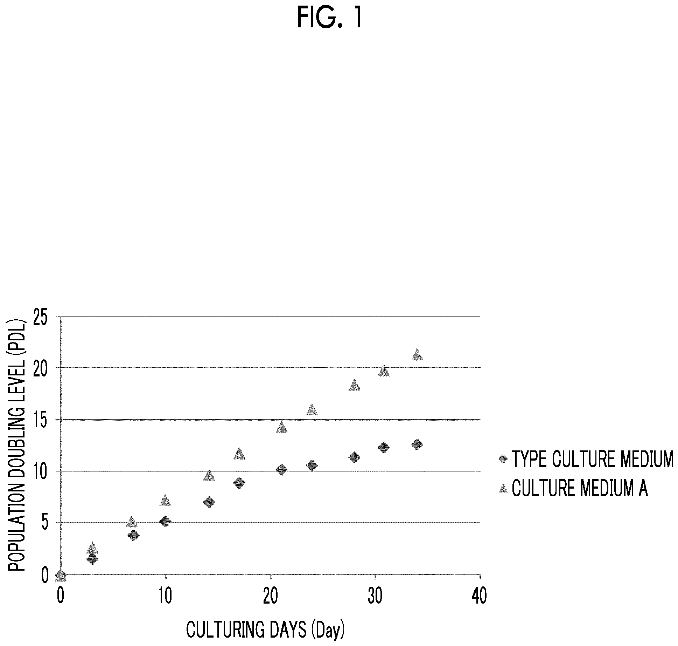

As a result of comparing the proliferation abilities, it was found that the proliferation ability of the MSC cultured in the culture medium A is significantly high as compared with that of the type culture medium. Fig. 1 shows a comparison of proliferation curves indicated by population doubling levels. The population doubling level (PDL) indicates the number of cell divisions and is calculated from the following expression. A proliferation curve was created by calculating and accumulating PDL for each passage.

Expression for calculating PDL: (Log (number of recovered cells) - Log (number of seeded cells))/Log2

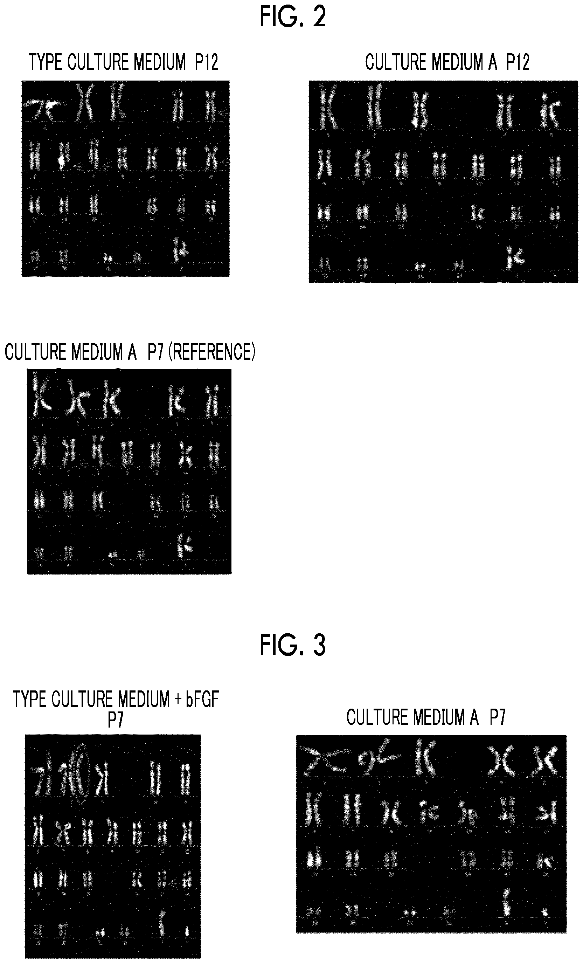

In addition, as a result of carrying out the karyotype analysis, it was found that karyotype abnormalities are observed in the MSC cultured in the type culture medium but no abnormalities are observed in the MSC cultured in the culture medium A. Table 2 and Fig. 2 show the results.

**[Table 2]**

| Culture medium | Number of chromosomes | Result | Karyotype | |
|---|---|---|---|---|
| Type culture medium P7 (cells subjected to 6 passages: reference) | No abnormalities in number of chromosomes | Karyotype abnormalities are present (structural abnormality) | 46,XY(7/8) | As shown on the left, karyotype abnormalities were detected in one cell out of eight cells. |
| | | | 46,XX,t(5;7;8)(q31 ;q31 ;q24)(1/8) | |
| Type culture medium P12 | No abnormalities in number of chromosomes | Karyotype abnormalities are present (structural abnormality) | 46,XY(4/5) | As shown on the left, karyotype abnormalities were detected in one cell out of eight cells. |
| | | | 46,XX,t(5;7;8)(q31 ;q31 ;q24)(1/5) | |
| Culture medium A P12 | No abnormalities in number of chromosomes | Normal | 46,XY(8/8) | As shown on the left, all cells were normal. |

### (2) Comparison of culturing and karyotype analysis of MSC using type culture medium + bFGF or culture medium A

Each of the frozen stocks (P5) of MSC that have been subjected to 4 passages in the type culture medium + bFGF of the lot: B012 and the culture medium A were thawed, subculturing was carried out twice in each culture medium, and the karyotype analysis of the cells of P7 (the cells subjected to 6 passages) was carried out.

As a result of comparing the proliferation abilities, it was possible to show the same degree of proliferation as that in the culture medium A, by adding bFGF to the type culture medium. Therefore, as a result of carrying out the karyotype analysis in the type culture medium + bFGF and the culture medium A, it was found that no karyotype abnormalities are observed in the MSC cultured in the culture medium A but karyotype abnormalities are observed in the MSC cultured in the type culture medium + bFGF. Table 3 and Fig. 3 show the results.

**[Table 3]**

| Culture medium | Number of chromosomes | | Result | Karyotype | Details |
|---|---|---|---|---|---|
| | | 46 | | | |
| Type culture medium + bFGF P7 | The number of chromosomes increased by one at a proportion of 10%, the trisomy of chromosome 2 was detected. Karyotype abnormalities are present. | | Abnormal (heteroploid) | 46,XY(7/8) | In the chromosome number analysis, 47 chromosomes were observed in two cells out of twenty cells, and the trisomy of the chromosome 2 was commonly observed, indicating the heteroploidity. In addition, the karyotyping revealed that seven cells out of eight cells have a normal karyotype of 46,XY, whereas the trisomy of the chromosome 2 and the chromosome gap (chtg) in chromosome 17 were observed in one cell, indicating the occurrence of karyotype abnormalities. |
| | | | | 47,XY,L2 chtg(17) (1/8) | |
| Culture medium A P7 | No abnormalities in chromosome number | | Normal | 46,XY(8/8) | No abnormalities in chromosome number in the chromosome number analysis. The karyotyping also revealed all eight cells have a normal karyotype of 46,XY. |

From the above results, it was found that the culturing method alone using the culture medium A can achieve both extremely remarkable proliferation improvement and normal karyotype.

### [Experiment B]

### (Materials and methods)

### (1) Cell

A human bone marrow mesenchymal stem cell or a porcine synovial mesenchymal stem cell was used in the experiment.

The human bone marrow mesenchymal stem cell was acquired and cultured by the method described in [Experiment A].

For the porcine synovial mesenchymal stem cell, the synovial tissue collected from the knee of a miniature pig (Fuji Micra Inc.) was shredded with scissors and immersed in 5.0 mL of a liberase solution. It is noted that as the liberase solution, a solution obtained by dissolving 5.0 mg of Liberase MNP-S (manufactured by F. Hoffmann-La Roche, Ltd.) in 5.0 mL of water for injection containing bovine fetal serum (NICHIREI BIOSCIENCES INC., model number: 174012) of a final concentration of 20% was used. The enzymatic reaction was carried out at 37°C for 3 hours. Then, the digested tissue solution was transferred to a 50 mL centrifuge tube through a cell strainer to be separated into two layers, and a lower layer was centrifuged at 400 g for 5 minutes. The supernatant was removed, and the obtained cell-concentrated suspension was suspended in the culture medium.

### (2) Basal medium

As Example, the culture medium (the culture medium A) described in [Experiment A] was used.

For comparison, αMEM (Thermo Fisher Scientific, Inc., model number: 12561-056) (the type culture medium) was used.

### (3) Culture vessel

As the culture vessel, a T-flask (TPP Techno Plastic Products AG, cell culture flask 150 cm² with filter cap, model number: 90151) or a 10-cell stack (Corning Inc., CellSTACK-10 Chamber, surface-treated for cell culture, model number: 3270) was used.

### (4) Culture of synovial mesenchymal stem cell

1% (v/v) of Antibiotic-Antimycotic (Thermo Fisher Scientific, Inc., model number: 15240-062) as an antibiotic as well as autologous serum in the culture of human bone marrow mesenchymal stem cells or bovine fetal serum (NICHIREI BIOSCIENCES INC., model number: 174012) in the culture of porcine synovial mesenchymal stem cells were added to each of the basal media described in (2) so that the final concentration of each serum was 10% to 20% (v/v), thereby obtaining a culture medium which was subsequently used for culturing synovial mesenchymal stem cells.

A cell suspension in which cells were suspended in the above-described culture medium at a predetermined concentration was prepared so that the cell seeding density and the amount of the culture medium after seeding in the culture vessel satisfied predetermined conditions, and a predetermined amount was seeded in the culture vessel described in (3). Here, the seeding density means the number of cells seeded per unit area of the culture surface, and the amount of the culture medium means the culture medium volume per unit area of the culture surface. The culture vessel after seeding was allowed to stand in an incubator at 37°C and in a 5% CO₂ atmosphere, and culturing was carried out. After carrying out culturing for a predetermined period from the seeding, the cells were detached and recovered from the culture vessel, and the number of cells was counted. The cell proliferation rate was calculated by dividing the number of recovered cells after the culture by the number of seeded cells.

### (Result)

### (Test 1) Comparison of proliferation rate due to difference in culture medium

Porcine synovial mesenchymal stem cells were cultured in the type culture medium to which 20% (v/v) of fetal bovine serum (FBS) was added. The cell seeding density was set to 1,000 or 1,500 cells/cm², the amount of the culture medium was set to 0.12 mL/cm², and a T-flask was used as the culture vessel. On the 12th day after the seeding, the cells were recovered, and each proliferation rate was calculated. Table 4 shows the relative proliferation rates in a case where the proliferation rate under the condition of the seeding density of 1,000 cells/cm² is set to 1.

Under the same conditions as in the case of the type culture medium to which FBS described above was added, the cells were cultured by changing only the culture medium to the culture medium A and recovered on the 12th day after the seeding, and the proliferation rate was calculated. Table 4 shows the relative proliferation rates in a case where the proliferation rate under the condition of the seeding density of 1,000 cells/cm² is set to 1 in a case where a type culture medium to which FBS was added was used.

In the culture of the porcine synovial mesenchymal stem cells, it was found that the culture medium A exhibits a high proliferation rate in a cell seeding density range of 1,000 to 1,500 cells/cm² as compared with the type culture medium.

**[Table 4]**

| Comparison 2 of relative proliferation rate due to difference in culture medium | | |
|---|---|---|
| | Seeding density [cells/cm²] | |
| | 1000 | 1500 |
| Type culture medium | 1.00 | 0.72 |
| Culture medium A | 1.21 | 0.92 |

### (Test 2) Comparison 1 of proliferation rate due to difference in amount of culture medium

Human bone marrow mesenchymal stem cells were cultured using the type culture medium and culture medium A.

In a comparative test 1, a culture medium obtained by adding 20% (v/v) of FBS to the type culture medium was used, and the amount of the culture medium was set to 0.12 mL/cm².

In a comparative test 2, a culture medium obtained by adding 20% (v/v) of FBS to the type culture medium was used, and the amount of the culture medium was set to 0.24 mL/cm².

In a test 3, a culture medium obtained by adding 20% (v/v) of FBS to the culture medium A was used, and the amount of the culture medium was set to 0.12 mL/cm².

In a test 4, a culture medium obtained by adding 20% (v/v) of FBS to the culture medium A was used, and the amount of the culture medium was set to 0.24 mL/cm².

Cells were recovered on the 12th day after the seeding under the conditions of the comparative tests 1 and 2 and the tests 3 and 4, and the proliferation rate was calculated. Table 5 shows the relative proliferation rate in a case where the proliferation rate of the comparative test 1 is set to 1.

It was found that, in both the type culture medium and the culture medium A as well, the proliferation rate is improved by increasing the amount of the culture medium.

**[Table 5]**

| Comparison of proliferation rate of human bone marrow mesenchymal stem cell due to difference in amount of culture medium | | | | |
|---|---|---|---|---|
| | Basal medium | Amount of culture medium [mL/cm²] | FBS concentration | Relative proliferation rate |
| Comparative test 1 | Type culture medium | 0.12 | 20% | 1.00 |
| Comparative test 2 | Type culture medium | 0.24 | 20% | 1.57 |
| Test 3 | Culture medium A | 0.12 | 20% | 1.35 |
| Test 4 | Culture medium A | 0.24 | 20% | 2.14 |

### (Test 3) Comparison 2 of proliferation rate due to difference in amount of culture medium

Porcine synovial mesenchymal stem cells were cultured using the type culture medium and culture medium A.

In a comparative test 11, a culture medium obtained by adding 20% (v/v) of FBS to the type culture medium was used, and the amount of the culture medium was set to 0.12 mL/cm².

In a comparative test 12, a culture medium obtained by adding 20% (v/v) of FBS to the type culture medium was used, and the amount of the culture medium was set to 0.20 mL/cm².

In a test 13, a culture medium obtained by adding 20% (v/v) of FBS to the culture medium A was used, and the amount of the culture medium was set to 0.12 mL/cm².

In a test 14, a culture medium obtained by adding 20% (v/v) of FBS to the culture medium A was used, and the amount of the culture medium was set to 0.20 mL/cm².

Cells were recovered on the 12th day after the seeding under the conditions of the comparative tests 11 and 12 and the tests 13 and 14, and the proliferation rate was calculated. Table 6 shows the relative proliferation rate in a case where the proliferation rate of the comparative test 1 is set to 1.

It was found that, in both the type culture medium and the culture medium A as well, the proliferation rate is improved by increasing the amount of the culture medium.

**[Table 6]**

| Comparison of proliferation rate of porcine synovial mesenchymal stem cell due to difference in amount of culture medium | | | | |
|---|---|---|---|---|
| | Basal medium | Amount of culture medium [mL/cm²] | FBS concentration | Relative proliferation rate |
| Comparative test 11 | Type culture medium | 0.12 | 20% | 1.00 |
| Comparative test 12 | Type culture medium | 0.20 | 20% | 2.15 |
| Test 13 | Culture medium A | 0.12 | 20% | 1.45 |
| Test 14 | Culture medium A | 0.20 | 20% | 2.59 |

### (Test 4) Evaluation of proliferation rate without increasing using amount of serum

Porcine synovial mesenchymal stem cells were cultured using the type culture medium and culture medium A together with basal medium.

In a test 21, a culture medium obtained by adding 20% (v/v) of FBS to the type culture medium was used, and the amount of the culture medium was set to 0.12 mL/cm².

In a comparative test 22, a culture medium obtained by adding 20% (v/v) of FBS to the culture medium A was used, and the amount of the culture medium was set to 0.12 mL/cm².

In a test 23, a culture medium obtained by adding 10% (v/v) of FBS to the culture medium A was used so that the total amount of the FBS to be used for the culture was the same as that of the test 21, and the amount of the culture medium was set to 0.24 mL/cm².

Cells were recovered on the 12th day after the seeding under the conditions of the test 21, the comparative test 22, and the test 23, and the proliferation rate was calculated. Table 7 shows the relative proliferation rate in a case where the proliferation rate of the comparative test 21 is set to 1.

In general, it is known that in a case where the serum concentration decreases, the proliferation rate decreases. This is conceived to be because the serum concentration contributes most to the cell proliferation rate. On the other hand, in the culture medium A, it was found that even in a case where the amount of serum used is the same, the proliferation rate is unexpectedly improved by increasing the amount of the basal medium and decreasing the serum concentration.

**[Table 7]**

| | Basal medium | Amount of culture medium [mL/cm²] | FBS concentration | Amount of FBS [mL/cm²] | Relative proliferation rate |
|---|---|---|---|---|---|
| Test 21 | Type culture medium | 0.12 | 20% | 0.024 | 1.00 |
| Comparative test 22 | Culture medium A | 0.12 | 20% | 0.024 | 1.44 |
| Test 23 | Culture medium A | 0.24 | 10% | 0.024 | 1.87 |

### (Test 5) Effect of serum concentration on proliferation rate

Human bone marrow mesenchymal stem cells were cultured using the type culture medium and culture medium A.

In a comparative test 31, a type culture medium to which 20% (v/v) of FBS to the type culture medium was added was used, and the amount of the culture medium was set to 0.12 mL/cm².

In a comparative test 32, a type culture medium to which 10% (v/v) of FBS was added so that the total amount of the FBS to be used for the culture was the same as that of the test 31 was used, and the amount of the culture medium was set to 0.24 mL/cm².

In a test 33, a culture medium A to which 10% (v/v) of FBS was used so that the total amount of the FBS to be used for the culture was the same as that of the test 31, and the amount of the culture medium was set to 0.24 mL/cm².

Table 8 shows the relative proliferation rates in a case where the proliferation rate after the culturing in each culture vessel for 12 days under the conditions of the comparative tests 31 and 32 and the test 33 are evaluated and the proliferation rate of the test 31 is set to 1.

It was found that in the type culture medium, the proliferation rate decreases as the serum concentration decreases even in a case where the amount of serum used is the same, whereas the proliferation rate is unexpectedly improved as compared with the type culture medium, by increasing the amount of the basal medium and decreasing the serum concentration in a case where the culture medium A is used as the basal medium. The fact that the proliferation rate decreases in a case where the serum concentration decreases, which has been confirmed in the type culture medium, is an event that is understood as a general phenomenon.

**[Table 8]**

| | Basal medium | Amount of culture medium [mL/cm²] | FBS concentration | Amount of FBS [mL/cm²] | Proliferation rate |
|---|---|---|---|---|---|
| Comparative test 31 | Type culture medium | 0.12 | 20% | 0.024 | 1.00 |
| Comparative test 32 | Type culture medium | 0.24 | 10% | 0.024 | 0.71 |
| Test 33 | Culture medium A | 0.24 | 10% | 0.024 | 1.31 |

### (Test 6) Effect of difference in culture vessel on proliferation rate

Porcine synovial mesenchymal stem cells were cultured using a T-flask and a 10-cell stack (CellSTACK-10 Chamber made of polystyrene, manufactured by Corning Inc.). As the culture medium, a culture medium obtained by adding 20% (v/v) of FBS to each of the type culture medium and the culture medium A was used, and the amount of the culture medium was set to 0.12 mL/cm².

In the comparative tests 41-1 and 41-2, the type culture medium was used, and the culture was carried out using each of the T-flask and the 10-cell stack was used. Table 9 shows the relative proliferation rates in the 10-cell stack in a case where the proliferation rate after the culturing in each culture vessel for 12 days is evaluated and the proliferation rate in the T-flask is set to 1.

In tests 42-1 and 42-2, the culture medium A was used, and the culture was carried out using each of the T-flask and the 10-cell stack.

Table 9 shows the relative proliferation rates in the 10-cell stack in a case where the proliferation rate after the culturing in each culture vessel for 12 days is evaluated and the proliferation rate in the T-flask is set to 1.

It was found that in a case where the culture vessel is changed from the T-flask to the 10-cell stack, the proliferation rate is decreased in the type culture medium, whereas the proliferation rate is improved in the culture medium A.

**[Table 9]**

| | Basal medium | Culture vessel | Relative proliferation rate |
|---|---|---|---|
| Comparative test 41-1 | Type culture medium | T flask | 1.00 |
| | | 10-cell stack | 0.79 |
| Comparative test 41-2 | Type culture medium | T flask | 1.00 |
| | | 10-cell stack | 0.98 |
| Test 42-1 | Culture medium A | T flask | 1.00 |
| | | 10-cell stack | 1.11 |
| Test 42-2 | Culture medium A | T flask | 1.00 |
| | | 10-cell stack | 1.38 |

### (Test 7) Effects of form of cap of culture vessel and presence or absence of forced aeration on proliferation rate

Porcine synovial mesenchymal stem cells were cultured in a 10-cell stack. As the culture medium, a culture medium obtained by adding 20% (v/v) of FBS to each of the type culture medium and the culture medium A was used, and the amount of the culture medium was set to 0.12 mL/cm². In each culture medium, a comparison was made between 10-cell stacks having two caps, where the two caps were standard filter caps or were changed from the standard filter caps to vent caps (Corning Inc., transfer cap for cell stack, model number; 3281). In addition, a comparison was made with a 10-cell stack in which the standard filter caps were changed to vent caps for the culture medium A and gas was forcibly aerated from the vent cap on one side. For the gas used for the forced aeration, a gas that was air containing 5% CO₂, which had been humidified (relative humidity: 100%), before introduction into the 10-cell stack, to the same level as in the incubator environment, and had been adjusted to a temperature of 37°C was used.

The cells were cultured for 12 days under each culture condition and recovered, and the proliferation rate was calculated. Table 10 shows the relative proliferation rates in a case where the proliferation result of the cells cultured in the T-flask using the same amount of the type culture medium is set to 1. It is noted that in the relative proliferation rate results in the table, those described as "culture cannot be continued" are those that have not reached the culturing for 12 days since it has been confirmed that the cells did not proliferate in the culture process.

In a case where the cap of the 10-cell stack was changed from the standard filter cap to the vent cap and the culture was carried out in the type culture medium, the proliferation was significantly reduced, and the culture could not be continued for a predetermined period. On the other hand, in a case where the culture medium A was used, the decrease in the proliferation rate could be suppressed as compared with the type culture medium even in a case where the standard filter cap was changed to the vent cap, and thus the culture could be continued. Further, it was found that in a case where the gas is forcibly aerated by changing the standard filter cap to the vent cap, the proliferation rate is remarkably improved.

**[Table 10]**

| | Basal medium | 10-cell stack cap | Forced aeration | Relative proliferation rate |
|---|---|---|---|---|
| Test 51 | Type culture medium | Standard filter cap | No | 0.84 |
| Test 52 | Type culture medium | Vent cap | No | Culture cannot be continued since cell are not proliferated (almost no cells are present) |
| Test 53 | Culture medium A | Standard filter cap | No | 1.28 |
| Test 54 | Culture medium A | Vent cap | No | 0.99 |
| Test 55 | Culture medium A | Vent cap | Aeration | 2.25 |

### (Test 8) Establishment of rat synovium-derived stem cell and effect of regenerating meniscus

The present test shows the ability of rat synovium-derived mesenchymal stem cells to regenerate the meniscus in a rat body.

A 6-week-old Lewis rat was used to establish synovium-derived mesenchymal stem cells. The synovial tissue collected under isoflurane anesthesia was reacted at 37°C for 2 hours in the basal medium A to which Fetal Bovine Serum (Gibco Cat. # 10270) had been added to a concentration of 20% and Antibiotic-Antimycotic (100X) (Thermo Fisher Scientific, Inc. Cat. # 15240062) had been added to a concentration of 1% and to which Collagenase V (Sigma Cat. # C9263) further had been added to 3.0 mg/mL. A cooled culture medium was added to stop the reaction, and the residual tissue was removed by being passed through a 40 µm cell strainer. The recovered cells were seeded in a cell culture flask of 75 cm² (Corning Inc., model number: 353136), followed by culturing at 37°C for 6 days at a CO₂ concentration of 5%. After culturing for 6 days, the culture medium in the flask was discarded and washing was carried out twice with phosphate buffered saline (PBS). Then, 5 mL of TrypLE (registered trade name) Express (Thermo Fisher Scientific, Inc. Cat. # 1260413) was added, and the cells were allowed to stand in an incubator at 37°C for 5 minutes and recovered as mesenchymal stem cells, followed by counting the number of cells using a live/dead cell autoanalyzer, Vi-CELL (BECKMAN COULTER, model number: 731050). The supernatant was discarded by centrifugation and replaced with COS-banker (Cosmo Bio Company, Limited, Cat. # COS-CFM01) to prepare a frozen stock of the cells.

A 10-week-old Lewis rat was used to prepare a meniscus injury model for evaluating the effect of regenerating the meniscus. In the method of transplanting meniscus injuries and mesenchymal stem cells, the skin of the knee joint part was incised under isoflurane anesthesia to expose the knee joint. The inner joint capsule under the patella was exposed, and a longitudinal incision was made with a scalpel to expose the cartilage in the distal end part of the femur. The medial meniscus was detached from the synovium to expose the medial meniscus, and about 2/3 of the whole thereof was excised. The patellar tendon and the synovium were sutured, and then the muscles were sutured. After the treatment for the meniscus injury, the cells were divided into two groups, a rat synovium-derived mesenchymal stem cell (rSMSC) group and a PBS group as a control. In the rSMSC group, for the mesenchymal stem cells to be administered, cells established from the synovium and frozen were wakened up and cultured for 8 days using a basal medium A containing 20% Fetal Bovine Serum (Gibco Cat. # 10270), 1% Antibiotic-Antimycotic (100 ×) (Thermo Fisher Scientific, Inc., Cat. # 15240062), the recovered cells of the number of 5 × 10⁶ cells were transplanted into the knee joint by piercing using an injection needle from the suture site in a direction perpendicular to the knee joint, and the skin was sutured. Postoperatively, all rats were returned to the cages and allowed to exercise, eat, and drink freely.

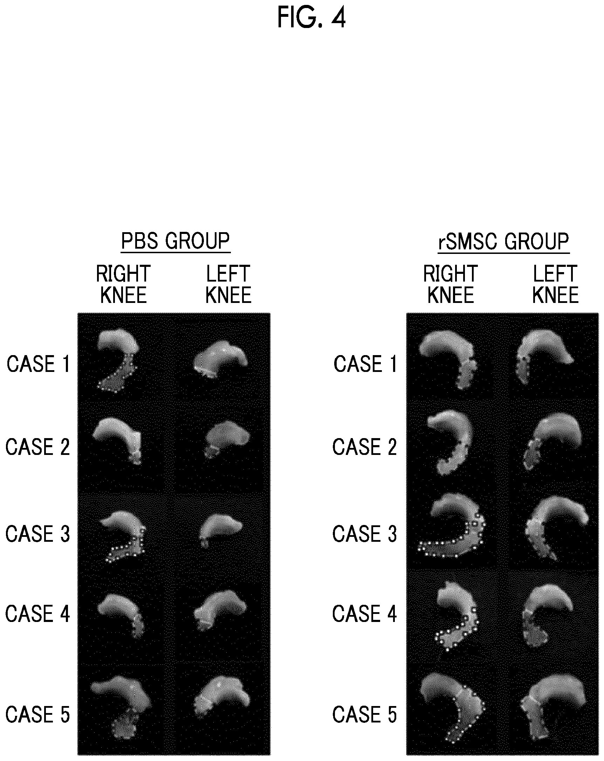

Animals were subjected to autopsy 3 weeks after the treatment. The animals were euthanized by cutting the descending aorta under isoflurane anesthesia. Then, the knee joint was removed to expose the meniscus, the medial meniscus was excised, and photographic images were taken. The images of the excised medial meniscus of both knee joints are shown in Fig. 4. A regenerated portion was specified in the image based on the difference in color tone and shape from the remaining meniscus and surrounded by a dotted line. In the PBS group, half of the meniscuses had a meniscus shape up to the middle segment; however, in the rSMSC group, there were many cases in which the meniscus was regenerated up to the anterior segment, from which the effect of regenerating the meniscus due to the rSMSC transplantation can be seen.

In order to quantitatively compare the effect of regenerating the meniscus between the rSMSC group and the PBS group, the area of the regenerated portion of the meniscus (inside the dotted line) was measured with Image J (version 1.52). The regenerated portion of the meniscus was visually determined based on the color tone and the shape of the meniscus, and the area was calculated using Expression 1.

### Expression 1:

Area of regenerated portion of meniscus (mm²) = number of pixels in area of regenerated portion of meniscus/number of pixels per 1 mm²

The average value, standard deviation, and statistical analysis of the areas of regenerated portions of each group were all carried out by Microsoft Excel 2007 (Microsoft Corp.). For the statistical analysis, Student's T-Test was carried out between the rSMSC group and the PBS group, and a significance level of less than 5% (P < 0.05) was regarded as having a difference.

Table 11 shows the values of the areas of the regenerated portions of the meniscuses in the rSMSC group and the PBS group. The area of the regenerated portion of the meniscus in the PBS group was 1.1 mm², whereas it was 2.0 mm² in the rSMSC group, which was approximately twice the regenerated area, whereby a statistically significant difference (P < 0.05) was observed.

**[Table 11]**

| Area of regenerated portion of meniscus (average value ± standard deviation | |
|---|---|
| Group | Area of regenerated portion of meniscus (mm²) |
| PBS group n = 5 (10 knees) | 1.1 ± 0.9 |
| rSMSC group n = 5 (10 knees) | 2.0 ± 0.8* |

| | |
|---|---|
| * P < 0.05 vs PBS group | |

## Claims

1. A method of producing a cell preparation for a joint medical treatment, the method comprising:
culturing a mesenchymal stem cell in a culture medium containing an ascorbic acid derivative, alanyl glutamine, and pyridoxine.

2. The method according to claim 1,
wherein the mesenchymal stem cell is a synovium-derived mesenchymal stem cell.

3. The method according to claim 1 or 2,
wherein the mesenchymal stem cell is of autologous origin.

4. The method according to any one of claims 1 to 3,
wherein the culture medium contains allogeneic serum.

5. The method according to any one of claims 1 to 4,
wherein the culture medium does not contain ascorbic acid.

6. The method according to any one of claims 1 to 5,
wherein the culture medium contains any one or more of biotin or lipoic acid.

7. The method according to any one of claims 1 to 6,
wherein the mesenchymal stem cell is cultured using a multilayer flask having five or more layers.

8. The method according to any one of claims 1 to 7, further comprising:
separating a suspension of a tissue containing mesenchymal stem cells into two layers of an upper layer and a lower layer; and
collecting the lower layer of the two layers,
wherein the mesenchymal stem cells contained in the lower layer are cultured in a culture medium containing an ascorbic acid derivative, alanyl glutamine, and pyridoxine.

9. A cell preparation for a joint medical treatment produced by the method according to any one of claims 1 to 8.

10. The cell preparation for a joint medical treatment according to claim 9,
wherein the cell preparation for a joint medical treatment does not contain an extracellular substrate or a scaffold.

11. The cell preparation for a joint medical treatment according to claim 9 or 10,
wherein the cell preparation for a joint medical treatment is a meniscus curing agent or an osteoarthritis curing agent.

12. The cell preparation for a joint medical treatment according to any one of claims 9 to 11,
wherein the mesenchymal stem cell is a human mesenchymal stem cell, and
the number of chromosomes of the mesenchymal stem cell is 46, where the chromosomes have a karyotype of pairs of chromosomes from chromosome 1 to chromosome 22 and, XX chromosomes or XY chromosomes.

13. A method of culturing a mesenchymal stem cell, the method comprising:
separating a suspension of a tissue containing mesenchymal stem cells into two layers of an upper layer and a lower layer; and
collecting the lower layer of the two layers; and
culturing the mesenchymal stem cells contained in the lower layer in a culture medium containing an ascorbic acid derivative, alanyl glutamine, and pyridoxine.

14. The method according to claim 13,
wherein the mesenchymal stem cell is a synovium-derived mesenchymal stem cell.

15. The method according to claim 13 or 14,
wherein the mesenchymal stem cell is of autologous origin.

16. The method according to any one of claims 13 to 15,
wherein the culture medium contains allogeneic serum.

17. The method according to any one of claims 13 to 16,
wherein the culture medium does not contain ascorbic acid.

18. The method according to any one of claims 13 to 17,
wherein the culture medium contains any one or more of biotin or lipoic acid.

19. The method according to any one of claims 13 to 18,
wherein the mesenchymal stem cell is cultured using a multilayer flask having five or more layers.

20. A cell preparation for a joint medical treatment, comprising the mesenchymal stem cell cultured by the method according to any one of claims 13 to 19.

21. The cell preparation for a joint medical treatment according to claim 20,
wherein the cell preparation for a joint medical treatment does not contain an extracellular substrate or a scaffold.

22. The cell preparation for a joint medical treatment according to claim 20 or 21,
wherein the cell preparation for a joint medical treatment is a meniscus curing agent or an osteoarthritis curing agent.

23. The cell preparation for a joint medical treatment according to any one of claims 20 to 22,
wherein the mesenchymal stem cell is a human mesenchymal stem cell, and
the number of chromosomes of the mesenchymal stem cell is 46, where the chromosomes have a karyotype of pairs of chromosomes from chromosome 1 to chromosome 22 and, XX chromosomes or XY chromosomes.
